# EUROPEAN PATENT APPLICATION

(11) **EP 2 702 923 A1**
(43) Date of publication of application: **05.03.2014**
(21) Application number: 12801168.1
(22) Date of filing: 11.06.2012
(51) Int. Cl.: A61B 1/00

(54) **ENDOSCOPE**

(30) Priority: 16.06.2011 JP 2011134397; 16.06.2011 JP 2011134398
(71) Applicant: Olympus Medical Systems Corp., Tokyo 151-0072 (JP)
(72) Inventor: MIYOSHI Hiroaki, Tokyo 151-0072 (JP); ISHIZAKI Ryosuke, Tokyo 151-0072 (JP)
(74) Representative: Schicker, Silvia
(86) International application number: PCT/JP2012/064893
(87) International publication number: WO 2012/173082

(57) **Abstract**

An endoscope includes a first function portion that is operated and bent with advancing and retracting movement of a first pulling member inserted in an insertion portion; a second function portion that is operated with advancing and retracting movement of a second pulling member inserted in the insertion portion; a first bending operation portion that is operated to rotate around a shaft to advance and retreat the first pulling member, and provided to an operation portion for operating and bending the first function portion; a second bending operation portion that is operated to rotate around a shaft to advance and retreat the second pulling member to operate the second function portion, and disposed to the operation portion with an insertion axis of the insertion portion being placed between the second bending operation and the first rotation operation knob; and an operation force amount adjusting portion provided to the second bending operation portion, and adjusting an operation force amount to operate and bend a second bending portion.

## Description

### Technical Field

The present invention relates to an endoscope that includes an operating wire in an insertion portion, besides a wire for bending operation of a bending portion, and includes, at an operation portion, a first operation knob that is rotationally operated when the wire for bending operation of the bending portion is operated to be pulled, and a second operation knob that is rotationally operated when the operating wire is operated to be pulled.

### Background Art

In recent years, in a medical field, an endoscope has been used which can perform observation or various kinds of therapeutic treatments by an elongated insertion portion being inserted into a body. At an insertion portion distal end side of an endoscope with a flexible insertion portion, a bending portion that is configured to be bent in, for example, a vertical and a lateral direction is provided.

The bending portion is configured to bend in the vertical and the lateral directions with a plurality of bending pieces being rotatably connected, for example. A bending wire that is a pulling member with a distal end fixed to the bending pieces that configure the bending portion is inserted through an inside of the insertion portion. The operation portion that is located at a proximal end of the insertion portion is provided with bending portion operating equipment for pulling the bending wire. A proximal end of the bending wire is fixed to, for example, a drum that configures the bending portion operating equipment.

According to the configuration, a surgeon operates the bending portion operating equipment with fingers of one hand that grasps the operation portion, and pulls a desired bending wire, and thereby the surgeon can operate and bend the bending portion in a direction intended by the surgeon.

Further, Japanese Patent Application Laid-Open Publication No. 2002-177198 shows an endoscope 2 that is favorable in operability and facilitates accurate observation and treatment. The endoscope 2 includes a first bending portion 24 and a second bending portion 25 in a bending portion 22 of an insertion portion 11. An operation portion 12a of the endoscope 2 includes a first bending operation portion 42 capable of independently operating and bending a first wire 34 that is extended from the first bending portion 24, and a second bending operation portion 44 capable of independently operating and bending a second wire 35 that is extended from the second bending portion 25. The first wire 34 is provided to connect to the first bending operation portion 42 through a first coil pipe 36 that is fixed to a vicinity of a distal end side of the second bending portion 25. The second wire 35 is provided to connect to the second bending operation portion 44 through an inside of a second coil pipe 37 that is fixed to a distal end side of a flexible tube portion 23.

However, the endoscope 2 disclosed in Japanese Patent Application Laid-Open Publication No. 2002-177198 is configured so that the first coil pipe 36 is inserted in the second bending portion 25. The inclusion of more components results in that, when the surgeon operates and bends the bending portion 22, the operation force amount of the second bending operation portion 44 operated to bend the second bending portion 25 is larger than the operation force amount of the first bending operation portion 42 operated to bend the first bending portion 24. This has caused the surgeon to have a sense of congruity concerning the difference between the operational feel of the second bending operation portion 44 and the operational feel of the first bending operation portion 42.

Further, Japanese Patent Application Laid-Open Publication No. 2003-220022 shows an endoscope excellent in both observation and treatment performance by sufficiently securing a total axial length in a bending portion to enhance insertion performance of an endoscope insertion portion, and realizing a bending shape easy to maneuver in a small space.

However, the endoscope of Japanese Patent Application Laid-Open Publication No. 2003-220022 is configured to require the surgeon to operate and bend the second bending portion by once releasing the hand holding the insertion portion from the insertion portion before operating the second bending operation portion. The surgeon was also unable to operate and bend the second bending portion with the hand holding the grasping portion.

The present invention was made in view of the above-described circumstances, and an object of the invention is to provide an endoscope which allows two types of rotation operation knobs provided to an operation portion for respectively pulling different pulling members to be easily operated with the fingers of the hand grasping the operation portion without releasing the hand from the insertion portion, which decreases the second bending portion operating force amount required to operate and bend the second bending portion including more internal components, and in which the second bending portion operation force amount required to bend the second bending portion is almost the same as a first bending portion operation force amount required to bend a first bending portion.

### Disclosure of Invention

### Means for Solving the Problem

An endoscope in one aspect of the present invention includes a first bending portion that is operated and bent with advancing and retracting movement of a first pulling member inserted in an insertion portion; a second bending portion that is operated with advancing and retracting movement of a second pulling member inserted in the insertion portion; a first bending operation portion that is operated to rotate around a shaft to advance and retreat the first pulling member, to operate and bend the first bending portion; a second bending operation portion that is operated to rotate around a shaft to advance and retreat the second pulling member to operate and bend the second bending portion; and an operation force amount adjusting portion provided to the second bending operation portion, and adjusting an operation force amount of the second bending portion to operate and bend the second bending portion.

### Brief Description of the Drawings

Fig. 1 to Fig. 8 relate to one embodiment of the present invention, and Fig. 1 is a view explaining a configuration of an endoscope including two kinds of rotation operation knobs;
Fig. 2 is a view of an operation portion including a first bending operation portion seen from an arrow Y2 direction of Fig. 1;
Fig. 3 is a view of the operation portion including a second bending operation portion seen from an arrow Y3 direction of Fig. 1;
Fig. 4 is a view explaining a state in which a first bending portion up/down knob is operated with a thumb of a hand grasping the operation portion, and a state in which a second bending portion up/down knob is operated;
Fig. 5 is a schematic view explaining a relation of a first rotation operation knob that is provided at an operation portion main body and a first pulling member that is inserted through an inside of the insertion portion, and a relation of a second rotation operation knob and a second pulling member inserted through the inside of the insertion portion;
Fig. 6 is a view of the second pulling member and a drive force transmitting mechanism seen from arrows Y6-Y6 direction of Fig. 5;
Fig. 7 is a sectional view taken along arrows Y7-Y7 line of Fig. 6;
Fig. 8 is a view explaining a relation of the second bending portion up/down knob, an operation force amount adjusting portion that also functions as the drive force transmitting mechanism, and a drum;
Fig. 9 and Fig. 10 relate to a configuration that regulates a rotation amount of a second bending portion up/down drum that is rotated by operation of the second rotation operation knob, and Fig. 9 is a view explaining a rotation angle restricting mechanism configured by an arc-shaped groove, a stopper and a rotation angle restricting pin;
Fig. 10 is a view explaining a disposition state of a stopper, and the operation portion including a knob index and an operation portion index;
Fig. 11A to Fig. 11C relate to a modification of the operation portion, and Fig. 11A is a view explaining a convex portion provided on one surface side of the operation portion where the second rotation operation knob is disposed;
Fig. 11B is a view of the operation portion including the second rotation operation knob and the convex portion seen from an arrow Y11B direction of Fig. 11A;
Fig. 11C is a view explaining an operation of the convex portion provided at the operation portion of the endoscope;
Fig. 12 is a view explaining a disposition example of a second bending portion up/down lock lever;
Fig. 13 is a view explaining another configuration example of the second rotation operation knob; and
Fig. 14 is a view explaining a configuration example of an endoscope in which two kinds of rotation operation knobs are provided at the second rotation operation knob.

### Best Mode for Carrying Out the Invention

Hereinafter, an embodiment of the present invention will be described with reference to the drawings.

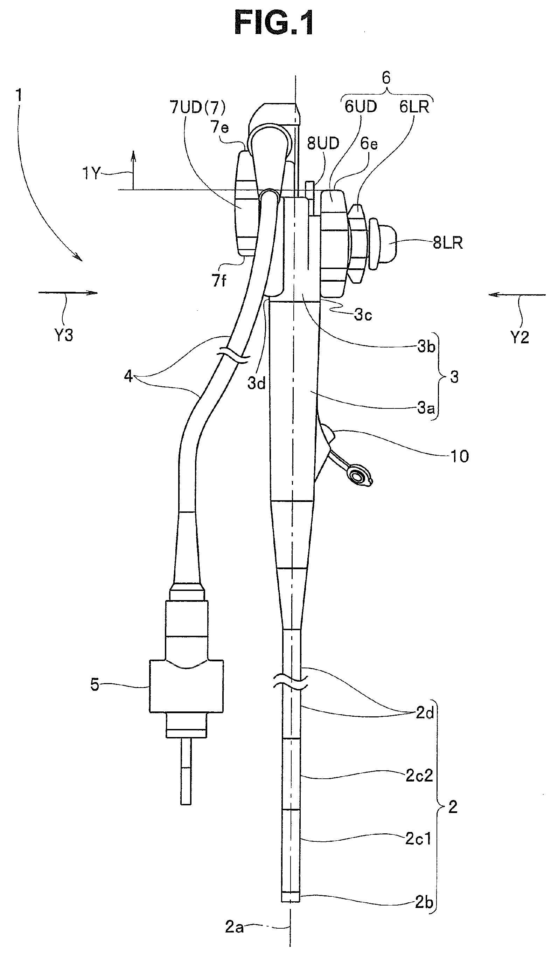

With reference to Fig. 1 to Fig. 10, one embodiment of an endoscope of the present invention will be described.

As shown in Fig. 1 to Fig. 3, an endoscope 1 includes an insertion portion 2 and an operation portion 3. The operation portion 3 is provided to connect to a proximal end side of the insertion portion 2, and also functions as a grasping portion. From a side portion of the operation portion 3, a flexible universal cord 4 is extended. A light guide connector 5 is provided at an end portion of the universal cord 4. The light guide connector 5 is detachably connected to a light source apparatus (not illustrated) that is an external apparatus. The operation portion 3 is provided on an extension line of an insertion axis 2a (see Fig. 1) of the insertion portion 2, at the proximal end side of the insertion portion 2.

The insertion portion 2 is configured by a rigid distal end portion 2b, a first bending portion 2c1, a second bending portion 2c2, and a flexible tube portion 2d having a long length and flexibility being provided to connect to one another in sequence from a distal end side.

The first bending portion 2c1 is a first function portion that is operated and bent with advancing and retracting movement of a first pulling member that will be described later, and is configured to be bendable in, for example, a vertical and a lateral directions. The second bending portion 2c2 is a second function portion that is operated and bent with advancing and retracting movement of a second pulling member that will be described later, and is configured to be bendable in, for example, the vertical direction.

As shown in Fig. 1 to Fig. 3, the operation portion 3 includes a grasping portion 3a and an operation portion main body 3b. The grasping portion 3a is a site which, for example, a surgeon grasps, and is provided at an insertion portion 2 side. The operation portion main body 3b is provided to connect to the grasping portion 3a. The operation portion main body 3b is provided with a first rotation operation knob 6 that configures a first bending operation portion, and a second rotation operation knob 7 that configures a second bending operation portion, with the insertion axis 2a therebetween.

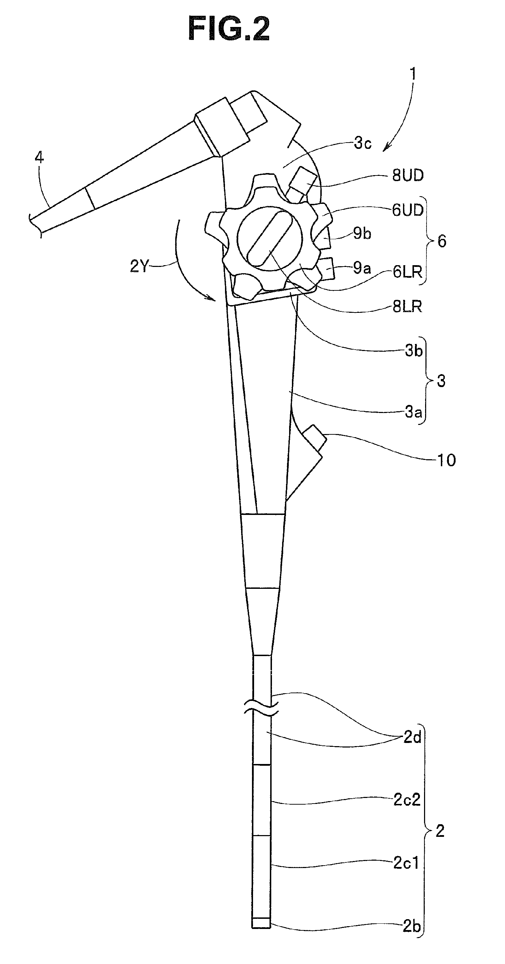

In the present embodiment, the first rotation operation knob 6 includes a first bending portion up/down knob 6UD, and a first bending portion left/right knob 6LR. The first bending portion up/down knob 6UD is a substantially ring-shaped knob for operating and bending the first bending portion 2c1 in the vertical direction. The first bending portion left/right knob 6LR is a knob for operating and bending the first bending portion 2c1 in the lateral direction. As shown in Fig. 1 and Fig. 2, the first bending portion up/down knob 6UD and the first bending portion left/right knob 6LR are provided to be overlaid onto a main body first side surface 3c.

Reference sign 8UD designates a first bending portion up/down lock lever, and retains a bending state of the first bending portion 2c1. Reference sign 8LR designates a first bending portion left/right lock lever, and retains a bending state of the first bending portion 2c1.

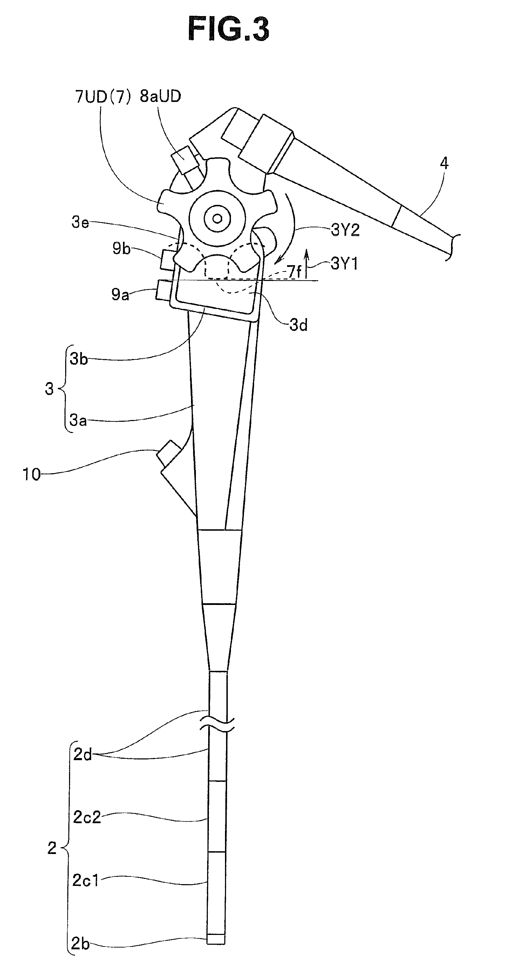

In the present embodiment, the second rotation operation knob 7 is a second bending portion up/down knob 7UD. The second rotation operation knob 7 is a knob for operating and bending the second bending portion 2c2 in the vertical direction. As shown in Fig. 1 and Fig. 3, the second bending portion up/down knob 7UD is provided at a main body second side surface 3d. Hereinafter, the second rotation operation knob 7 will be described as the second bending portion up/down knob 7UD.

In the present embodiment, the main body first side surface 3c and the main body second side surface 3d are opposed surfaces.

Note that as shown in Fig. 3, an air/water feeding button 9a and a suction button 9b are provided to protrude on a main body third side surface 3e. The air/water feeding button 9a is a fluid control switch that controls supply of a gas and a liquid, and the suction button 9b is a fluid control switch that controls suction. Reference sign 10 designates a treatment instrument insertion port. The treatment instrument insertion port 10 communicates with a treatment instrument channel that also functions as a fluid channel which is inserted through the inside of the insertion portion 2. A treatment instrument such as a biopsy forceps is introduced into a body via the treatment instrument insertion port 10, a treatment instrument channel and the like.

As shown in Fig. 1, the second bending portion up/down knob 7UD is provided at the operation portion 3 by a position at which the knob 7UD is placed, or an outside diameter dimension of the knob 7UD being properly set, and by a most proximal end disposition position 7e of the second bending portion up/down knob 7UD being set so as to be located at the proximal end side of the insertion axis 2a as shown by an arrow 1Y from a most proximal end disposition position 6e of the first bending portion up/down knob 6UD. According to the placing state, a most distal end disposition position 7f of the second bending portion up/down knob 7UD is not disposed at the proximal end side of the insertion axis 2a from the most proximal end disposition position 6e of the first bending portion up/down knob 6UD.

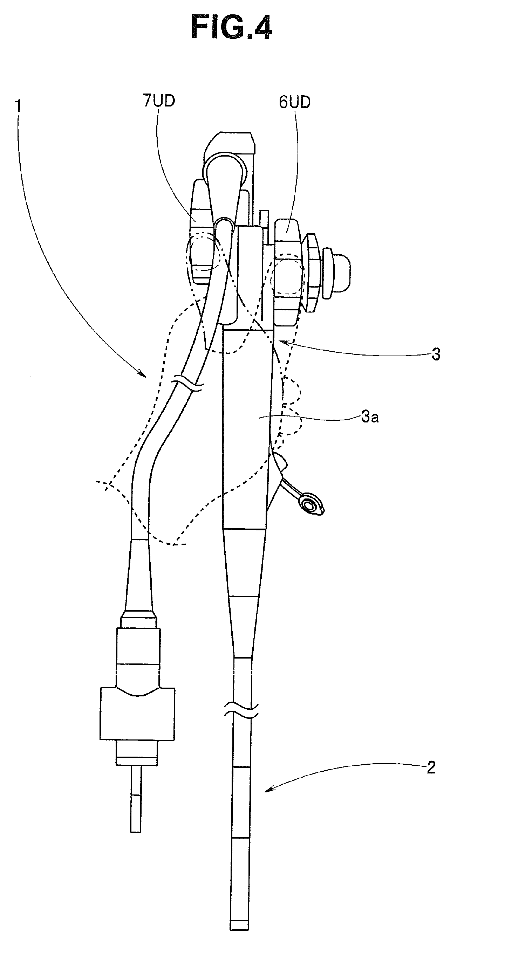

According to the configuration, a thumb of a hand that grasps the operation portion 3 is moved as shown by the broken line, or the two-dot chain line of Fig. 4, and operation of the first bending portion up/down knob 6UD, operation of the first bending portion left/right knob 6LR, or operation of the second bending portion up/down knob 7UD can be properly performed. In other words, operation of the knobs 6UD, 6LR and 7UD which are provided at the operation portion 3 of the endoscope 1 can be performed without the hand grasping the operation portion 3 being taken off the operation portion 3. Accordingly, the surgeon does not have to take the other hand grasping the insertion portion 2 off the insertion portion 2.

Note that as shown in Fig 3, in the operation portion 3, the most distal end disposition position 7f of the second bending portion up/down knob 7UD is placed so as to be located at the proximal end side of the insertion axis 2a as shown by an arrow 3Y1 from a proximal end position of the air/water feeding button 9a. As a result, in a state in which the operation portion 3 is grasped, operation of the air/water feeding button 9a, or operation of the suction button 9b can be smoothly performed with any one of a forefinger, a middle finger and a ring finger of the hand grasping the operation portion 3.

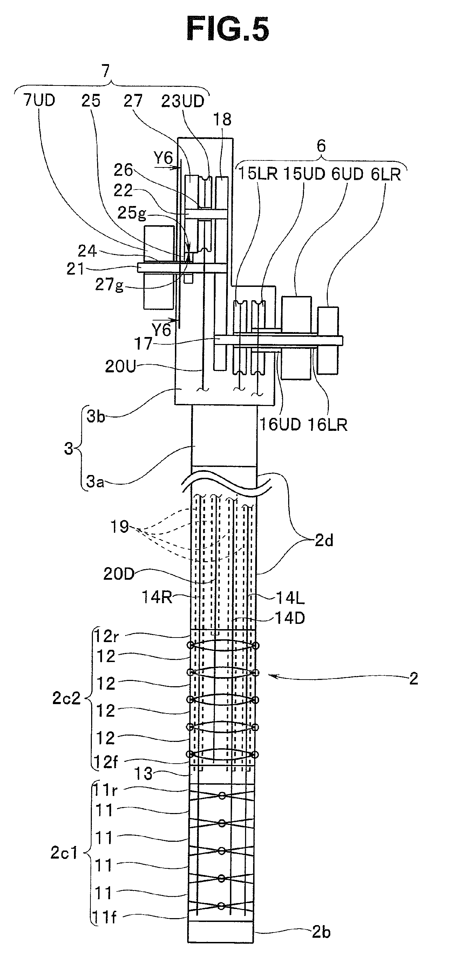

As shown in Fig. 5, the first bending portion 2c1 of the insertion portion 2 is configured to be bent in the vertical and the lateral directions by, for example, a plurality of bending pieces 11, 11, ... being rotatably connected to one another. A first bending portion distal end bending piece 11f that configures the first bending portion 2c1 is connected to a proximal end side of the distal end portion 2b, and a first bending portion proximal end bending piece 11r is connected to a distal end side of a connection piece 13.

To positions corresponding to an up, a down, a right and a left of the first bending portion distal end bending piece 11f, distal ends of the first bending portion wires 14 corresponding to the up, the down, the left and the right directions that are first pulling members are fixed by blazing or the like. The first bending portion wires 14 are inserted through an inside of the first bending portion 2c1, an inside of the second bending portion 2c2, an inside of the flexible tube portion 2d and an inside of the grasping portion 3a, and are guided into the operation portion main body 3b.

The first bending portion wires 14 are inserted through insides of coil pipes 19 that are provided in the second bending portion 2c2 and in the flexible tube portion 2d.

Among the first bending portion wires 14, a proximal end of an up wire 14 (not illustrated) and a proximal end of a down wire 14d are fixed to a first bending portion up/down drum 15UD, and a proximal end of a left wire 14L and a proximal end of a right wire 14R are fixed to a first bending portion left/right drum 15LR.

Note that the first bending portion up/down drum 15UD and the first bending portion left/right drum 15LR, and the rotation operation knob 6 configure a first bending operation portion.

The first bending portion up/down drum 15UD is fixed to the other end of an up/down shaft 16UD to one end of which the first bending portion up/down knob 6UD is fixed. Accordingly, the first bending portion up/down drum 15UD integrally rotates with rotation in a clockwise direction or in a counterclockwise direction of the first bending portion up/down knob 6UD. The first bending portion left/right drum 15LR is fixed to the other end of a left/right shaft 16LR to one end of which the first bending portion left/right knob 6LR is fixed. Accordingly, the first bending portion up/down drum 15UD integrally rotates with rotation of the first bending portion left/right knob 6LR.

According to the configuration, when the surgeon bends, for example, the first bending portion 2c1 in the up direction, the surgeon rotates the first bending portion up/down knob 6UD in an arrow 2Y direction of Fig. 2. Thereupon, with the rotation of the first bending portion up/down knob 6UD, the first bending portion up/down drum 15UD rotates in the same direction, and the up wire not illustrated, which is fixed to the first bending portion up/down drum 15UD, is pulled, while the down wire 14D is slackened. As a result, with the rotation of the first bending portion up/down knob 6UD, the first bending portion 2c1 bends in the up direction intended by the surgeon.

Note that reference sign 17 designates a first bending portion shaft body, and is fixed upright to a partition plate 18, which is provided inside the operation portion main body 3b. The knobs 6UD and 6LR rotate in the clockwise direction and the counterclockwise direction around the axis with respect to the first bending portion shaft body 17.

The second bending portion 2c2 of the insertion portion 2 is configured to bend in the vertical direction by, for example, a plurality of bending pieces 12, 12, ... being rotatably connected to one another. A second bending portion distal end bending piece 12f that configures the second bending portion 2c2 is connected to a proximal end side of the connection piece 13, and a second bending portion proximal end bending piece 12r is connected to a distal end side of the flexible tube portion 2d.

To positions corresponding to an up and a down of the second bending portion distal end bending piece 12f, distal ends of second bending portion wires 20 corresponding to the up and the down directions, that are second pulling members are respectively fixed by brazing or the like. The second bending portion wires 20 are inserted through the inside of the second bending portion 2c2, the inside of the flexible tube portion 2d and the inside of the grasping portion 3a, and are guided into the operation portion main body 3b.

The second bending portion wires 20 are inserted through the insides of the coil pipes 19 provided in the flexible tube portion 2d.

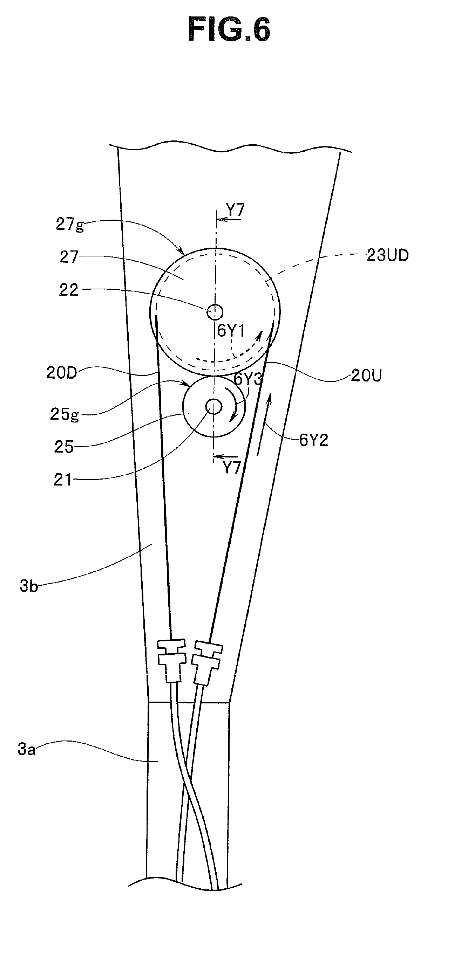

As shown in Fig. 6, a proximal end of an up wire 20U that is the second bending portion wire 20 guided into the operation portion 3 and a proximal end of a down wire 20D are fixed to preset positions of a second bending portion up/down drum 23UD that is a pulling portion.

Note that the second bending portion up/down knob 7UD and the second bending portion up/down drum 23UD configure a second bending operation portion.

Further, in the present embodiment, the up wire 20U and the down wire 20D cross each other in, for example, the grasping portion 3a, and the disposition positions in the insertion portion 2 and the disposition positions in the operation portion 3 are reversed.

According to the configuration, the second bending portion up/down drum 23UD is rotated counterclockwise as shown by an arrow 6Y1 in Fig. 6, whereby the up wire 20U is pulled as shown by an arrow 6Y2 and the second bending portion 2c2 bends in the up direction.

Here, with reference to Fig. 5 to Fig. 7, a relation of the second bending portion up/down knob 7UD and the second bending portion up/down drum 23UD will be described.

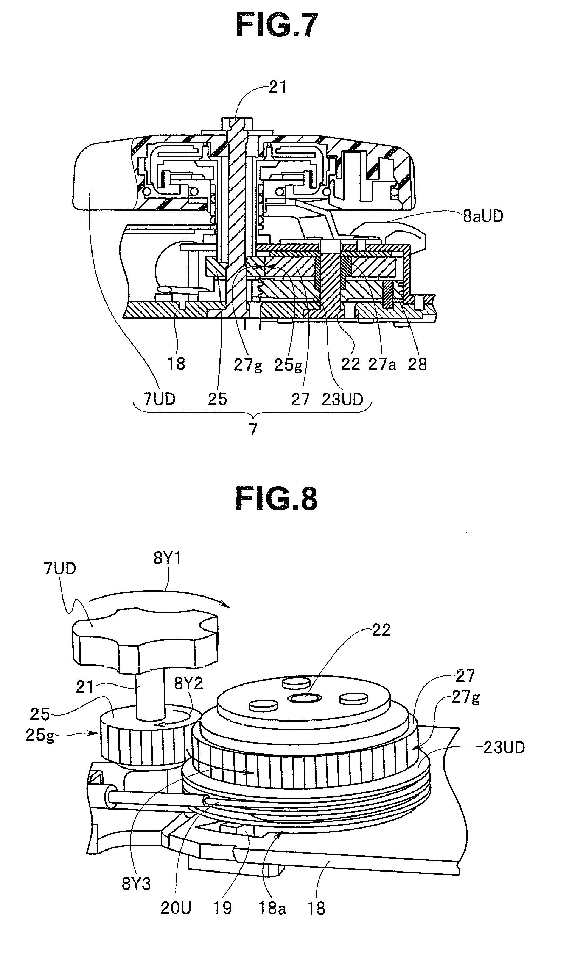

As shown in Fig. 5 and Fig. 7, the second bending portion up/down knob 7UD that configures the second bending operation portion is provided at a second bending portion first shaft body (hereinafter, abbreviated as a first shaft body) 21. In contrast with this, the second bending portion up/down drum 23UD is provided at a second bending portion second shaft body (hereinafter, abbreviated as a second shaft body) 22. The second bending portion up/down knob 7UD rotates in the clockwise direction and the counterclockwise direction around the axis with respect to the first shaft body 21.

The second bending operation portion is provided with a first gear 25 and a second gear 27 that is, for example, a gear train that is an operation force amount adjusting portion that also functions as a drive force transmitting mechanism portion that transmits a rotation force of the second bending portion up/down knob 7UD to the second bending portion up/down drum 23UD. That is, the second bending operation portion is configured by including the second bending portion up/down knob 7UD, the second bending portion up/down drum 23UD, the first gear 25 and the second gear 27.

Note that the second bending portion up/down drum 23UD and the second gear 27 are integrally fixed in advance via a gear portion shaft body 27a.

The first shaft body 21 and the second shaft body 22 are fixedly provided upright with respect to the partition plate 18 respectively. At a preset position of the first shaft body 21, a first rotation shaft 24 is rotatably provided. At a preset position of the second shaft body 22, a second rotation shaft 26 is rotatably provided.

To one end side of the first rotation shaft 24, the second bending portion up/down knob 7UD is integrally fixed, and to the other end side, the first gear 25 is integrally fixed. To a preset position of the second rotation shaft 26, the second bending portion up/down drum 23UD to which the second gear 27 is integrally fixed is fixed. A tooth portion 25g included by the first gear 25 and a tooth portion 27g included by the second gear 27 are meshed with each other.

The number of teeth of the first gear 25 and the number of teeth of the second gear 27 are properly set with consideration given to a second bending portion operating force amount with which the second bending portion up/down knob 7UD is operated when the second bending portion 2c2 is bent. In the present embodiment, at least the first gear 25 is set as a small gear, and the second gear 27 is set as a large gear with the number of teeth being larger than the number of teeth of the first gear 25. According to the configuration, the second bending portion operating force amount at a time when the surgeon operates and bends the second bending portion 2C2 by operating the second bending portion up/down knob 7UD becomes smaller as compared with an operation force amount with which the second bending portion is directly operated and bent as the first bending portion up/down knob 6UD due to the operation of the second bending portion up/down knob 7UD.

Further, when the surgeon bends, for example, the second bending portion 2c2 in the up direction, the surgeon rotates the second bending portion up/down knob 7UD in an arrow 3Y2 (arrow 8Y1 in Fig. 8) direction of Fig. 3, whereby with rotation of the second bending portion up/down knob 7UD, the first gear 25 rotates in an arrow 8Y2 (arrow 6Y3 in Fig. 6) direction of Fig. 8 which is the same direction.

As described above, the tooth portion 25g of the first gear 25 and the tooth portion 27g of the second gear 27 are in a meshed state. Accordingly, with rotation of the first gear 25, the second gear 27 rotates in an arrow 8Y3 (arrow 6Y1 in Fig. 6) direction of Fig. 8 which is an opposite direction.

Further, as described above, the second gear 27 and the second bending portion up/down drum 23UD are integrally fixed to the second rotation shaft 26. Therefore, the second bending portion up/down drum 23UD also rotates in the arrow 8Y3 (arrow 6Y1 of Fig. 6) direction of Fig. 8.

As a result, the up wire 20U is pulled as shown by an arrow 6Y2 as described above, while the down wire 20D is slackened, and the second bending portion 2c2 is bent in the up direction.

Note that reference sign 8aUD (also described in Fig. 3) of Fig. 7 designates a second bending portion up/down lock lever that retains a bending state of the second bending portion 2c2.

As above, the operation portion 3 that is connectively provided at a proximal end side of the insertion portion 2 is provided with the first bending portion up/down knob 6UD and the first bending portion left/right knob 6LR as the first rotation operation knob 6, and the second bending portion up/down knob 7UD as the second rotation operation knob 7 with the insertion axis 2a therebetween. As a result, the surgeon can properly perform operation of the first bending portion up/down knob 6UD, operation of the first bending portion left/right knob 6LR and operation of the second bending portion up/down knob 7UD by moving the thumb, the forefinger, the middle finger or the ring finger of the grasping hand without taking the hand off the operation portion 3 in the state in which the surgeon grasps the operation portion 3.

As above, in the second bending operation portion, such a configuration is adopted that transmits the rotation of the second bending portion up/down knob 7UD to the second bending portion up/down drum 23UD via the first gear 25 and the second gear 27. In addition, the number of teeth of the first gear 25 is set to be smaller than the number of teeth of the second gear 27. As a result, reduction of the second bending portion operating force amount at the time of the second bending portion 2c2 in which the coil pipe 19 is contained being operated and bent can be realized.

In the embodiment described above, the number of teeth of the first gear 25 is set to be smaller than the number of teeth of the second gear 27. However, more specifically, the number of teeth of the first gear 25 that is a small gear, and the number of teeth of the second gear 27 that is a large gear are properly set after the first bending portion operating force amount with which the first bending portion 2c1 is bent by operation of the first bending portion up/down knob 6UD is measured in advance. In other words, a gear ratio of the first gear 25 and the second gear 27 are properly set.

As a result, the second bending portion operating force amount with which the second bending portion is bent by operation of the second bending portion up/down knob 7UD is set to substantially correspond to the first bending portion operating force amount that is, for example, an operation force amount desired by the user, and the endoscope can be configured, in which the sense of operation of the first rotation operation knob 2c1 and the sense of operation of the second rotation operation knob 2c2 are substantially equal to each other.

Further, in the aforementioned embodiment, the drive force transmitting mechanism includes the first gear 25 and the second gear 27. However, the operation force amount adjusting portion that is also used as the drive force transmitting mechanism portion is not limited to a gear train, but may be a combination of a belt and pulleys, a combination of a chain and sprockets, or the like. In the configurations, diameter dimensions of a first pulley and a second pulley, or the number of teeth of a first sprocket and the number of teeth of a second sprocket are properly set as described above, whereby an optimal sense of operation can be obtained.

Furthermore, a diameter dimension of the second bending portion is set to be larger, and an internal density in the second bending portion is reduced, or the thickness of a bending cover configuring the second bending portion is formed to be thin, whereby reduction of the second bending portion operating force amount at the time of bending the second bending portion may be achieved.

Further, in the aforementioned embodiment, the second bending portion 2c2 is used as the second function portion which is provided at the insertion portion 2. However, the second function portion of the insertion portion 2 which is operated by advancement and retraction of the second pulling member is not limited to the second bending portion, but may be, for example, a variable rigidity mechanism, a zoom mechanism, a forceps raising mechanism or the like.

The variable rigidity mechanism is provided in a so-called a variable flexibility endoscope, and makes rigidity of the flexible tube which is connectively provided at the bending portion which is the first function portion switchable. The variable rigidity mechanism is configured by including a coil placed in the flexible tube portion, and the variable rigidity wire which is the second pulling member which changes the rigidity of the coil. The variable rigidity wire is connected to the second rotation operation knob, whereby the surgeon can perform the bending operation of the bending portion and the operation of changing the rigidity of the flexible tube portion by moving the thumb, the forefinger, the middle finger or the ring finger of the hand grasping the operation portion 3 without taking the hand off the insertion portion 2.

A zoom mechanism is provided in an observation optical system, and is contained in a distal end portion provided at the distal end of the bending portion that is the first function portion. The zoom mechanism is configured by including a moving lens frame, and an operation wire. The moving lens frame is provided at an objective lens system of the observation optical system to freely advance and retract. The operation wire is the second pulling member which causes the moving lens frame to perform advancing and retracting movement. The operation wire is connected to the second rotation operation knob, whereby the surgeon can perform bending operation of the bending portion and a switching operation of wide-angle observation or enlargement observation of an observation image by moving the thumb, the forefinger, the middle finger or the ring finger of the hand grasping the operation portion 3 without taking the hand off the insertion portion 2.

The forceps raising mechanism is contained in the distal end portion provided at the distal end of the bending portion which is the first function portion. The forceps raising mechanism is configured by including a forceps raising table and a raising wire. The forceps raising table is rotatably disposed at a preset position of the distal end portion. The raising wire changes a rising angle of the forceps raising table. The raising wire is connected to the second rotation operation knob, whereby the surgeon can perform bending operation of the bending portion and the switching operation of a lead-out direction of the treatment instrument by moving the thumb, the forefinger, the middle finger or the ring finger of the hand grasping the operation portion 3 without taking the hand off the insertion portion 2.

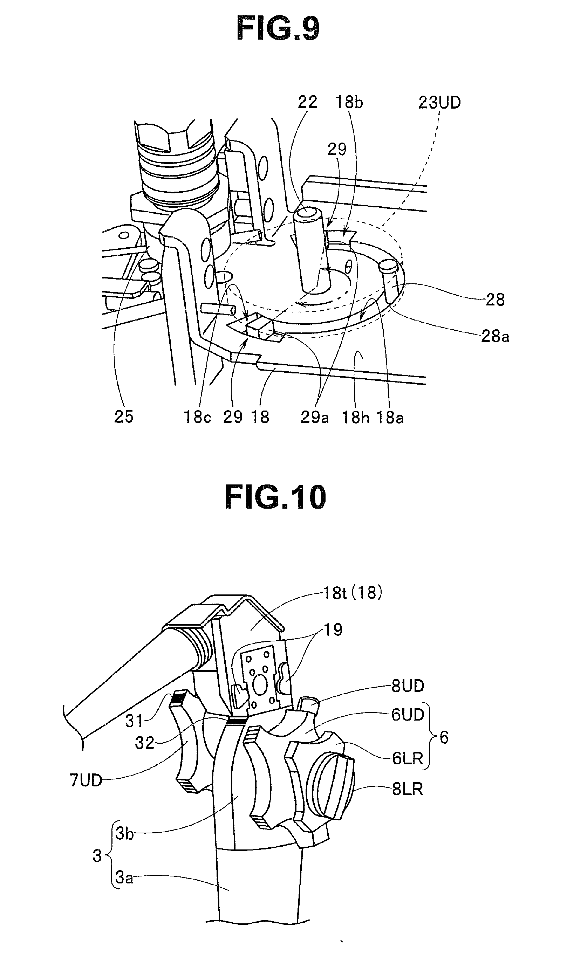

With reference to Fig. 9 and Fig. 10, a rotation angle regulating mechanism of the second rotation operation knob will be described.

Fig. 9 is a view explaining the rotation angle regulating mechanism configured by an arc-shaped groove, a stopper and a rotation angle regulating pin, and Fig. 10 is a view explaining a disposition state of the stopper, and the operation portion including a knob index and an operation portion index.

At the second bending portion up/down drum 23UD, a rotation angle regulating pin (see reference sign 28 of Fig. 7 and Fig. 9) which defines a rotation amount of the second bending portion up/down drum 23UD is fixedly provided. The rotation angle regulating pin 28 includes a protruded portion 28a which is protruded from one surface of the second bending portion up/down drum 23UD. The protruded portion 28a is movably disposed at an arc-shaped groove 18a which is formed on a surface 18h of the partition plate 18.

Stopper disposition holes 18b and 18c are formed at one end side and the other end side of the arc-shaped groove 18a. In the stopper disposition holes 18b and 18c, stoppers 29 each including an abutment surface 29a on which the rotation angle regulating pin 28 abuts are disposed. The stopper disposition holes 18b and 18c are closed by large diameter portions 29b of the stoppers 29 which are disposed at a back surface 18t side of the partition plate 18 as shown in Fig. 10.

According to the configuration, the protruded portion 28a of the rotation angle regulating pin 28 which is fixedly provided at the second bending portion up/down drum 23UD abuts on the abutment surface 29a of the stopper 29 which is placed in the first stopper disposition hole 18b, whereby the up wire 20U is brought into a maximum pulled state. The protruded portion 28a of the rotation angle regulating pin 28 abuts on the abutment surface 29a of the stopper 29 which is placed in the second stopper disposition hole 18c, whereby the down wire 20D is brought into the maximum pulled state.

A moving range of the rotation angle regulating pin 28 is properly set with the bending angle of the second bending portion 2c2 taken into consideration. Note that the moving range of the rotation angle regulating pin 28 is an angle θ from the abutment surface 29a of the stopper 29 which is placed in the first stopper disposition hole 18b to the abutment surface 29a of the stopper 29 which is placed in the second stopper disposition hole 18c.

As above, the rotation angle regulating pin 28 is fixedly provided at the second bending portion up/down drum 23UD, and the rotation angle regulating pin 28 is movably disposed in the arc-shaped groove 18a with the stoppers 29 respectively provided at both ends. As a result, the rotation amount of the second bending portion up/down drum 23UD can be regulated to be less than one rotation.

Note that reference sign 31 designates the knob index, and reference sign 32 designates the operation portion index. When the knob index 31 and the operation portion index 32 correspond to each other, a neutral position is established. In the present embodiment, when the second function portion is the second bending portion 2c2, the second bending portion 2c2 is set to be in a substantially linear state in the neutral position. Further, when the second function portion is a variable rigidity mechanism, the flexible tube portion is set to be in the state having the highest flexibility in the neutral position. Further, when the second function portion is a zoom mechanism, the observation optical system is set to be in a wide-angle observation state in the neutral position. Further, when the second function portion is a forceps raising mechanism, the forceps raising table is set to be in a state before rising in the neutral position.

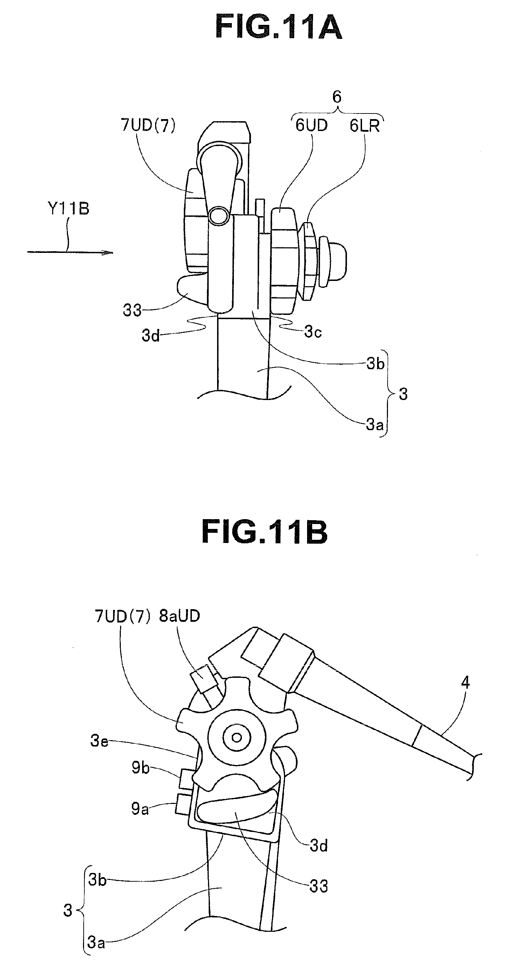

Fig. 11A to Fig. 11C are modifications of the operation portion.

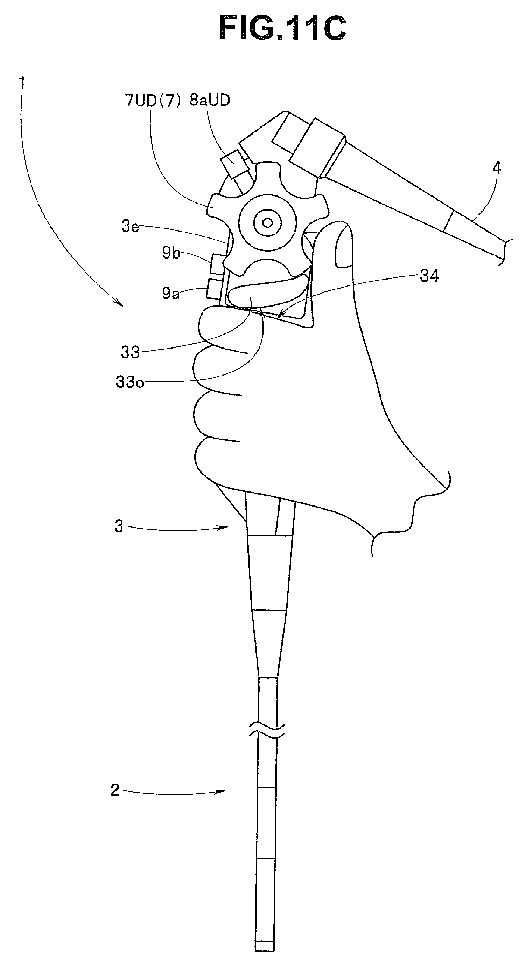

Fig. 11A is a view explaining a convex portion provided at one surface side of the operation portion where the second rotation operation knob is disposed. Fig. 11B is a view of the operation portion including the second rotation operation knob and the convex portion seen from an arrow Y11B direction of Fig. 11A. Fig. 11C is a view explaining an operation of the convex portion provided at the operation portion of the endoscope.

As shown in Fig. 11A and Fig. 11B, an endoscope 1A of the present embodiment is provided with a convex portion 33 having a preset shape and a preset height at a preset position of the second side surface 3d of the main body of the operation portion 3.

As shown in Fig. 11B, the convex portion 33 is formed at a grasping portion 3a side of the operation portion main body 3b, and covers and wraps an outer circumferential side of the grasping portion 3a side of the second bending portion up/down knob 7UD.

According to the configuration, the forefinger or the like of the surgeon can be prevented from touching the second bending portion up/down knob 7UD and rotating the second bending portion up/down knob 7UD as shown in Fig. 11C.

That is, when the surgeon performs operation of the air/water feeding button 9a, or operation of the suction button 9b with any finger of the forefinger, the middle finger and the ring finger of the grasping hand without taking the hand off the operation portion 3 in the state in which the surgeon is grasping the operation portion 3, the finger is prevented from touching the second bending portion up/down knob 7UD.

According to the endoscope 1, in the grasping state of Fig. 11C, for example, the surgeon disposes a side portion (reference sign 34) of the forefinger to cause the forefinger to abut on an outer side surface 33o of the convex portion 33, and thereby the surgeon can hold the operation portion 3 without dropping the operation portion 3 with a very small grasping force without firmly grasping the operation portion 3. That is, the convex portion 33 also functions as a holding portion that holds the endoscope 1.

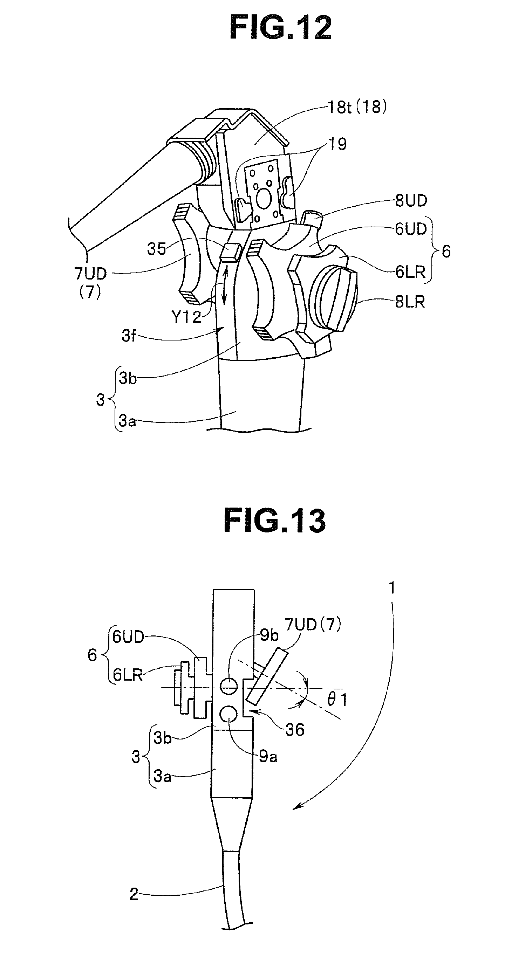

Note that in the aforementioned embodiment, the second bending portion up/down lock lever 8aUD is made to have a lever structure similar to the first bending portion up/down lock lever 8UD. However, the second bending portion up/down lock lever is not limited to the lever structure, but may be a slide member 35 slidable with respect to a fourth side surface 3f of the main body as shown by an arrow Y12 of Fig. 12.

Further, in the aforementioned embodiment, the first bending portion shaft body 17, the first shaft body 21 and the second shaft body 22 are provided fixedly to the partition plate 18 in the upright state. However, the endoscope 1 may be configured in such a manner that a center axis 21a of the first shaft body 21 crosses a center axis 17a of the first bending portion shaft body 17 at an angle θ1 as shown in Fig. 13.

Reference sign 36 of Fig. 13 designates a relief portion provided at the operation portion 3. The relief portion 36 prevents hindrance of rotation of the second bending portion up/down knob 7UD by the second bending portion up/down knob 7UD abutting on the operation portion 3. The other configurations are the same as the configurations of the aforementioned embodiment, the same members are assigned with the same reference signs, and the description thereof will be omitted.

According to the configuration, the operation and the effect similar to the above description can be obtained.

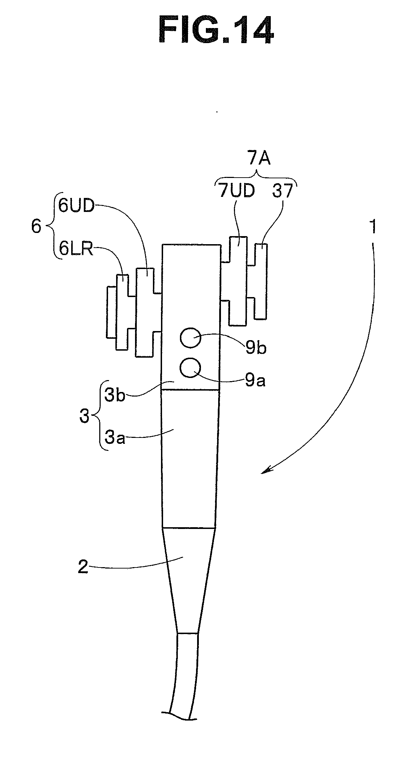

Further, in the aforementioned embodiment, one knob is provided at the second rotation operation knob 7, and by the knob, the second pulling member configuring the second function portion is configured to be moved to advance and retract. However, when the endoscope is an endoscope 1A that includes, for example, a variable rigidity mechanism (not illustrated) in addition to the first bending portion 2c1 and the second bending portion 2c2, the endoscope may be configured such that a variable rigidity operation knob 37 for operating a variable rigidity mechanism is provided at a second rotation operation knob 7A, in addition to 7UD that performs bending operation of the second bending portion 2c2, as shown in Fig. 14.

According to the configuration, the surgeon can properly perform operation of the first bending portion up/down knob 6UD, operation of the first bending portion left/right knob 6LR, operation of the second bending portion up/down knob 7UD and operation of the variable rigidity operation knob 37 by moving the thumb of the hand grasping the operation portion 3 without taking the hand off the insertion portion 2.

Note that the endoscope including the aforementioned zoom mechanism, or the endoscope including a forceps raising mechanism, in place of the variable rigidity mechanism, may be adopted.

Note that the present invention is not limited only to the embodiment described above, and can be carried out by being variously modified within the range without departing from the gist of the present invention.

The present application is filed claiming the priority of Japanese Patent Applications No. 2011-134397 filed in Japan on June 16, 2011, No. 2011-134398 filed in Japan on June 16, 2011, and the above described disclosure is incorporated by reference in the present description, claims and drawings.

## Claims

1. An endoscope, comprising:
a first bending portion which is a first function portion that is operated and bent with advancing and retracting movement of a first pulling member inserted in an insertion portion;
a second function portion that is operated with advancing and retracting movement of a second pulling member inserted in the insertion portion;
a first bending operation portion that is operated to rotate around a shaft to advance and retract the first pulling member, and provided to an operation portion for operating and bending the first bending portion;
a second bending operation portion that is operated to rotate around a shaft to advance and retreat the second pulling member to operate the second function portion, and disposed to the operation portion with an insertion axis of the insertion portion being placed between the second bending operation and the first rotation operation knob; and
an operation force amount adjusting portion provided to the second bending operation portion, and adjusting an operation force amount to operate and bend the second bending portion.

2. The endoscope according to claim 1,
the operation force amount adjusting portion sets a second bending portion operating force amount with which the second bending portion is operated and bent by operating the second rotation operation knob which configures the second bending operation portion to be smaller than an operation force amount needed for directly pulling the second pulling member.

3. The endoscope according to claim 2,
wherein the operation force amount adjusting portion adjusts the second bending portion operating force amount with which the second bending portion is operated and bent by operating the second rotation operation knob, and makes the second bending portion operating force amount substantially equal to a first bending portion operating force amount at a time of the first bending portion being operated and bent by operating the first rotation operation knob which configures the first bending operation portion, or smaller than the first bending portion operating force amount.

4. The endoscope according to any one of claims 1 to 3,
wherein the operation force amount adjusting portion is configured by at least two gears.

5. The endoscope according to claim 4,
wherein the operation force amount adjusting portion adjusts the second bending portion operating force amount by setting of a gear ratio of a first gear and a second gear.

6. The endoscope according to claim 1,
wherein in a configuration in which the first rotation operation knob which configures the first bending operation portion and the second operation knob which configures the second bending operation portion are placed at an operation portion that also functions as a grasping portion including the insertion shaft provided at the insertion portion proximal end,
a most proximal end disposition position of the second rotation operation knob is disposed at a proximal end side of the insertion shaft from at least a most proximal end disposition position of the first rotation operation knob.

7. The endoscope according to claim 6,
wherein in a configuration including at the operation portion a plurality of fluid control switches that control supply or suction of a fluid,
a most distal end disposition position of the second rotation operation knob is disposed at the proximal end side of the insertion shaft from at least a fluid control switch located at a distal end side among the plurality of fluid control switches.

8. The endoscope according to claim 1, further comprising:
a drive force transmitting portion that is connected to the second rotation operation knob and transmits a rotation operation that is inputted in the second rotation operation knob as a rotation force, and a pulling portion that advances and retracts the second pulling member by the rotation force.

9. The endoscope according to claim 8,
wherein the drive force transmitting portion transmits the rotation force by a gear train configured by at least a first gear and a second gear.

10. The endoscope according to claim 9,
when the second function portion is a second bending portion provided to the insertion portion, that performs bending operation with advancing and retracting movement of the second pulling member,
the second pulling member crosses in any one of the operation portion and the insertion portion.
